# EUROPÄISCHE PATENTSCHRIFT

(11) **EP 1 819 275 B1**
(45) Veröffentlichungstag und Bekanntmachung des Hinweises auf die Patenterteilung: **20.07.2011**
(21) Anmeldenummer: 05803622.9
(22) Anmeldetag: 21.10.2005
(51) Int. Cl.: A61B 5/154, A61M 5/34

(54) **PROBENRÖHRCHEN ZUR AUFNAHME VON KÖRPERFLÜSSIGKEITEN, INSBESONDERE BLUT**
SAMPLE TUBE FOR RECEIVING BODY FLUIDS, PARTICULARLY BLOOD
TUBE DE PRELEVEMENT DESTINE A CONTENIR DES LIQUIDES CORPORELS, EN PARTICULIER DU SANG

(30) Priorität: 23.11.2004 DE 102004056655
(43) Veröffentlichungstag der Anmeldung: 22.08.2007
(73) Patentinhaber: Sarstedt AG & Co., 51588 Nümbrecht (DE)
(72) Erfinder: SARSTEDT, Walter, 51588 Nümbrecht (DE)
(74) Vertreter: Grosse, Wolf-Dietrich Rüdiger
(86) Internationale Anmeldenummer: PCT/DE2005/001887
(87) Internationale Veröffentlichungsnummer: WO 2006/056150

(56) Entgegenhaltungen:
- EP-A- 1 426 069
- WO-A-03/055390
- US-A- 3 542 024

## Beschreibung

Die Erfindung betrifft ein Probenröhrchen zur Aufnahme von Körperflüssigkeiten, insbesondere Blut, mit einer Verschlusskappe, die einen Dom mit einer in der Domspitze angeordneten, durchstechbaren Membrane für eine auf den Dom aufsteckbare, eine Kanüle oder Doppelkanüle tragende Führungshülse besitzt, und das mit einem farbigen Notfall-Kennzeichnungsmittel bestückbar ist.

Bei einer aus der DE 30 49 503 C bekannten Blutentnahmevorrichtung besitzt die das Entnahmeröhrchen an seinem vorderen Ende abschließende Kappe einen zylindrischen, in axialer Richtung vorstehenden Dom. Der Dom ist an seinem vorderen Ende durch einen durchstechbaren Verschlussstopfen/eine Membrane abgeschlossen, der /die auf einer mit einer Mittelbohrung versehenen Stirnplatte des Doms aufliegt und von einem am vorderen Ende umgebördelten Kragen gehalten wird. Die Verschlusskappe wird vorzugsweise auf das Probenröhrchen aufgeschraubt, alternativ aufgesteckt. Die rohrförmige Führungshülse, die an ihrem vorderen Ende in einem Halter eine doppelendige, beiderseits mit einer Schneidkante versehene Kanüle trägt, deren aus der Führungshülse vorstehendes Ende zur Einführung in eine Vene dient, während ihr hinteres Ende so weit in die Führungshülse hineinragt, dass es beim Ansetzen der Führungshülse an das Probenröhrchen die Membrane/den Verschlussstopfen durchsticht, ist axial verschiebbar und drehbar auf dem Dom angeordnet. Das hintere, in die Führungshülse hineinragende Kanülenende wird von einem sackartigen Schlauch (Ventilgummi) solcher Länge eingehüllt, dass die Schneidkante des hinteren Kanülenendes bei gestrecktem Schlauch noch nicht dessen Boden berührt. Zur Verbindung der Doppelkanüle mit dem Dom ist letzterer bei der bekannten Blutentnahmevorichtung mit einem seitlich vorstehenden Haltenocken versehen, dem Axialschlitze in der Führungshülse zugeordnet sind. Mittels des in einen der über den Umfang verteilten Axialschlitze eingeführten Haltenocken lässt sich eine bajonettverschlussartige Drehverriegelung der in lockerer Passung auf dem Dom sitzenden Führungshülse der Doppelkanüle erreichen.

Bei einer anderen bekannten Ausführung einer Verschlusskappe (vgl. EP 0 818 296 B1) zum Aufstecken oder-schrauben auf das offene Ende eines Probenröhrchens sind zur Verriegelung der Führungshülse drei um 90° versetzt zueinander angeordnete Haltenocken vorgesehen.

Bei Notfällen, in denen das entnommene Blut sehr schnell einer Analyse zugeführt werden muss, ist es in der Praxis bekannt, das entsprechende Probenröhrchen mit einem Notfall-Kennzeichnungsmittel zu versehen, so dass für das Bedienungspersonal sofort und unmittelbar die Notfallsituation und große Eilbedürftigkeit erkennbar ist. Hierzu ist es seit vielen Jahren üblich, ein solches Probenröhrchen mit einer farbigen Etikette zu versehen oder auf den Dom der Verschlusskappe eine farbige Membrankappe aufzustecken. Sowohl bei der einen als auch der anderen dieser Maßnahmen liegt der Nachteil vor, dass die farbliche Notfallcodierung eines Probenröhrchens bei insbesondere zusammen mit zahlreichen, nicht als Notfall gekennzeichneten Probenröhrchen in einen gemeinsamen Ständer eingestellten Probenröhrchen doch nicht so deutlich zu erkennen ist, wie es der Notfall erfordert. Denn die Probenröhrchen mit Etiketten verlieren sich in der Ständer-Aufnahme ebenso wie die Membrankappen, die nur bis zu den Haltenocken des Doms aufgesteckt sind und folglich nur eine kleinflächige Signalwirkung bieten. Hinzu kommt noch, dass ein mit einer farbigen Membrankappe gekennzeichnetes Probenröhrchen zur Analyse zwar einer Direktadaption zugänglich ist, jedoch die in das Probenröhrchen eindringende Probennehmernadel einerseits die Membrane der Kappe und andererseits die Membrane des Doms der Verschlusskappe durchdringen muss. Die hierzu erforderliche Kraftaufbringung, weil ein großer Widerstand überwunden werden muss, kann dazu führen, dass die Membrankappe zerstört wird.

Der Erfindung liegt daher die Aufgabe zugrunde, ein gattungsgemäßes Probenröhrchen ohne die genannten Nachteile zu schaffen, das insbesondere die Notfall-Kennzeichnung sicher zu erkennen gibt und außerdem eine größere Betriebssicherheit bei der Analyse der entnommenen Proben-Körperflüssigkeit mit sich bringt.

Diese Aufgabe wird erfindungsgemäß dadurch gelöst, dass das Notfall-Kennzeichnungsmittel als ein den Dommantel mit von oben freier Zugänglichkeit zur Domspitze umhüllender Notfall-Signalfarbenring ausgebildet ist. Hiermit lassen sich mehrere Vorteile gleichzeitig erreichen. Es liegt nämlich durch den weitestgehend den gesamten Dom umschließenden Signalfarbenring eine größtmögliche Signalfläche vor, die nahezu der gesamten Umfangsfläche des vorhandenen Doms entspricht. Der Signalfarbenring besitzt hierzu einen im Durchmesser vergrößerten Hülsen-Endabschnitt, so dass er sich bis über den/die Haltenocken des Doms stülpen lässt, während sich hingegen ein Kopfabschnitt des Signalfarbenringes eng an den Außenumfang des Doms anlegt.

Der vorzugsweise aus einem Leuchtfarbenmaterial bestehende Signalfarbenring endet zweckmäßigerweise unmittelbar vor der in der Regel konischen Domspitze. Das freiliegende Konusende der Domspitze trägt dazu bei, dass die zur Analyse der entnommenen Probe erforderliche Zentrierbarkeit des Probenröhrchens erhalten bleibt. Schließlich ist das mit einem erfindungsgemäßen Signalfarbenring gekennzeichnete Probenröhrchen einer Direkt-Adaption möglich, ohne dass zwei Verschlussstopfen oder Membranen durchstochen werden müssen.

Weitere Einzelheiten und-Merkmale-der Erfindung ergeben sich aus dem Patentanspruch und der nachfolgenden Beschreibung eines in den Zeichnungen dargestellten Ausführungsbeispiels der Erfindung. Es zeigen:
- Figur 1: als Einzelheit einer Blutentnahmevorrichtung in einer Gesamtansicht ein Probenröhrchen mit auf dem Dom der Verschlusskappe aufge- stecktem Notfall-Signalfarbenring; und
- Figur 2: eine Darstellung wie zuvor mit demgegenüber längsgeschnittenem Signalfarbenring.

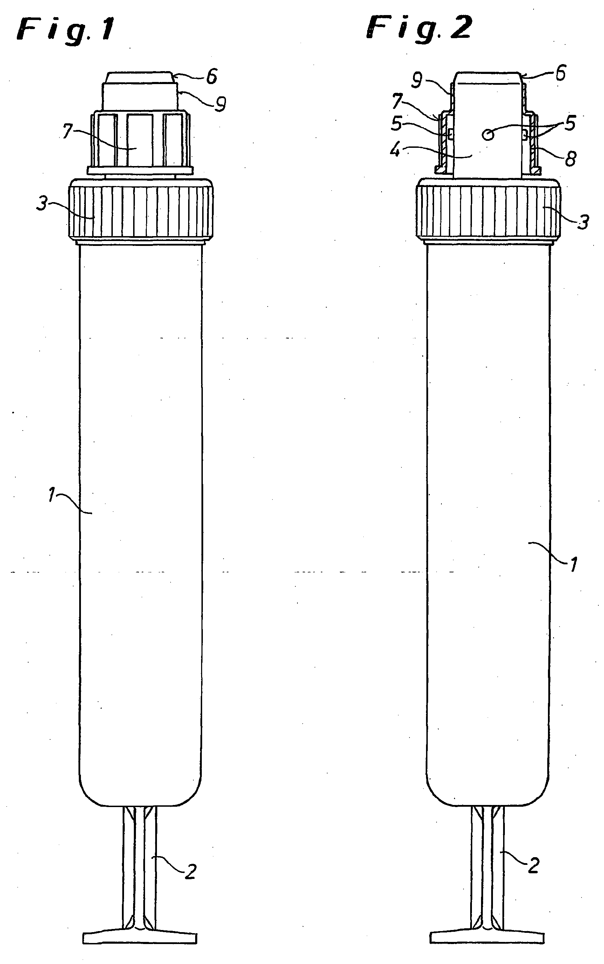

Von einer Blutentnahmevorrichtung ist in den Figuren 1 und 2 in einem vergrößerten Maßstab ein zylindrisches Probenröhrchen 1 gezeigt, in dem eine Kolbenstange 2 mit einem Kolben luftdicht verschiebbar angeordnet ist. An seinem vorderen Ende ist das Probenröhrchen 1 durch eine im Ausführungsbeispiel aufschraubbare Verschlusskappe 3 verschlossen. Von dieser steht in axialer Richtung ein Dom 4 vor, der zur Verriegelung mit einer nicht dargestellten, eine Kanüle oder Doppelkanüle tragende Führungshülse mit Haltenocken 5 (vgl. Figur 2) ausgebildet ist. In der konischen Domspitze 6 ist eine Membrane oder ein Stopfen (nicht dargestellt) vorgesehen.

Zur Kennzeichnung des Probenröhrchens 1 als Notfall ist auf den Dom 4 als Kennzeichnungsmittel ein Signalfarbenring 7, z.B. in einer Leuchtfarbe, aufgesteckt. Dieser ist ebenfalls einstückig durch Spritzgießen aus Kunststoff hergestellt und besitzt einen bezogen auf den Dom im Durchmesser größeren Hülsen-Endabschnitt 8 und einen sich nach oben hin verjüngenden Kopfabschnitt 9. Beim Aufstecken des Signalfarbenrings 7 auf den Dom 4 gleitet der Hülsen-Endabschnitt 8 über die Haltenocken 5 und reicht bis auf den Aufschraubteil der Verschlusskappe 3, während sich der Kopfabschnitt 9 bis zum Konusende erstreckt und eng an den Außenumfang des Doms 4 anlegt. Der Signalfarbenring 7 umhüllt damit den gesamten Dommantel und bietet somit eine größtmögliche Signalfläche, die auch dann leicht zu erkennen ist, wenn ein solches Probenröhrchen 1 mit nicht für den Notfall gekennzeichneten Probenröhrchen in einem Ständer abgestellt wird.

Das frei bleibende Konusende der Domspitze 6 gewährleistet die gute Zentrierbarkeit des solchermaßen als Notfall gekennzeichneten Probenröhrchens 1. Außerdem ist die in der Domspitze 6 angeordnete Membrane trotz der großflächigen Signalkennzeichnung von oben frei zugänglich, so dass das für den Notfall gekennzeichnete Probenröhrchen 1 sogleich einer ungefährdeten Direkt-Adaption in einem Analysegerät zugänglich ist.

## Patentansprüche

1. Probenröhrchen (1) zur Aufnahme von Körperflüssigkeit, insbesondere Blut, mit einer Verschlusskappe (3), die einen Dom (4) mit einer in der Domspitze (6) angeordneten, durchstechbaren Membrane, Stopfen oder dergleichen für eine auf den Dom aufsteckbare, eine Kanüle oder Doppelkanüle tragende Führungshülse besitzt, und das mit einem Notfall-Kennzeichnungsmittel bestückbar ist,
**dadurch gekennzeichnet,**
**dass** das Notfall-Kennzeichnungsmittel als ein den Dommantel mit von oben freier Zugänglichkeit zur Domspitze (6) umhüllender Notfall-Signalfarbenring (7) ausgebildet ist.

## Claims

1. A sample tube (1) for receiving body fluid, in particular blood, comprising a sealing cap (3) which has a dome (4) with a piercable membrane, stopper or the like arranged in the tip (6) of said dome for a guide sleeve carrying a cannula or double cannula that can be placed on said dome, and which can be equipped with an emergency means of identification,
**characterised in that**
the emergency means of identification is designed as an emergency signal coloured ring (7) surrounding the dome cover with free access from above to the tip (6) of said dome.

## Revendications

1. Tube de prélèvement (1) destiné à recevoir un fluide corporel, en particulier du sang, comportant un bouchon de fermeture (3) qui possède une broche (4) avec une membrane, un bouchon ou similaire perforable disposé(e) dans la pointe de la broche (6) pour une douille de guidage portant une canule ou une double canule, pouvant être enfichée sur la broche, et qui est muni d'un moyen d'identification en cas d'urgence,
**caractérisé en ce que**
le moyen d'identification en cas d'urgence est formé comme une bague de couleur de signal d'urgence (7) entourant l'enveloppe de broche avec un accès libre depuis le haut jusqu'à la pointe de la broche (6).
